(19) Europäisches Patentamt
European Patent Office
Office européen des brevets

(11) **EP 4 088 697 A1**

(12) **EUROPEAN PATENT APPLICATION**

(43) Date of publication:
**16.11.2022 Bulletin 2022/46**

(21) Application number: **21172970.2**

(22) Date of filing: **10.05.2021**

(51) International Patent Classification (IPC):
**A61F 13/15** (2006.01)    **A61F 13/532** (2006.01)
**A61F 13/53** (2006.01)

(52) Cooperative Patent Classification (CPC):
**A61F 13/5323; A61F 13/15658;**
A61F 2013/530131; A61F 2013/53051;
A61F 2013/5307

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **The Procter & Gamble Company
Cincinnati, OH 45202 (US)**

(72) Inventor: **JACKELS, Hans Adolf
53881 Euskirchen (DE)**

(74) Representative: **P&G Patent Germany
Procter & Gamble Service GmbH
Sulzbacher Straße 40
65824 Schwalbach am Taunus (DE)**

(54) **PROCESS FOR FORMING COMPOSITE ABSORBENT MATERIAL AND COMPOSITE ABSORBENT MATERIAL MADE BY THE PROCESS**

(57)    The present invention relates to a process for forming a composite absorbent structure, the process comprising the steps of:

advancing a nonwoven web in a machine direction, wherein the nonwoven web has a first surface and an opposing second surface, wherein the first and second surfaces define a substrate comprising void spaces, and wherein the nonwoven web comprises from 20% to 100% by weight of superabsorbent fibres;

distributing superabsorbent particles on to the first surface of the nonwoven web; and drawing at least some of the superabsorbent particles into void spaces of the nonwoven web substrate to form the composite absorbent structure.

The present invention further relates to composite absorbent structure comprising: a first surface and an opposing second surface, wherein the first and second surfaces define a substrate comprising void spaces, and wherein the nonwoven web comprises from 20% to 100% by weight of superabsorbent fibres; and superabsorbent particles; wherein the superabsorbent particles are distributed heterogeneously in the vertical direction between the first and second surfaces.

In another embodiment the present invention relates to absorbent article comprising a substantially liquid permeable topsheet, a substantially liquid impermeable backsheet, and a composite absorbent structure as described above, wherein the composite absorbent structure lies between the topsheet and the backsheet, and wherein the first surface is located on the side of the absorbent article closest to the topsheet, so that larger superabsorbent particles are predominantly distributed closest to a body side of the absorbent article.

Processed by Luminess, 75001 PARIS (FR)

**Description**

FIELD OF THE INVENTION

The invention relates to processes for forming composite absorbent material and to absorbent structures and their use in personal hygiene absorbent articles. The absorbent structures may in particular be used in baby diapers, adult incontinence or feminine hygiene products.

BACKGROUND OF THE INVENTION

Absorbent articles for personal hygiene such as disposable baby diapers, training pants for toddlers or adult incontinence undergarments, are designed to absorb and contain body exudates, in particular urine. These absorbent articles comprise several layers providing different functions, typically including a topsheet, a backsheet and an absorbent core in-between, among other layers.

The absorbent core should be able to absorb and retain the exudates for a prolonged amount of time, for example overnight for a diaper, minimize re-wet to keep the wearer dry, and avoid soiling of clothes or bed sheets. Absorbent cores have typically comprised a blend of comminuted wood pulp cellulose fibers with superabsorbent polymers (SAP) particles, also called absorbent gelling materials (AGM), as absorbent material.

Absorbent cores without fluff cellulose fibers (also called "airfelt-free" cores) have been more recently proposed. The SAP particles may be for example enclosed within discrete pockets formed between two substrates, for example WO95/11654. It has also been proposed to immobilize SAP particles with a microfibrous adhesive network to a nonwoven substrate by an adhesive, for example WO2008/155699.

More recently, airfelt-free cores have been disclosed comprising a high loft, fibrous, central layer with SAP particles at least partially distributed within this high loft layer, for example WO2016/106021. SAP particles are applied on each side of the high loft nonwoven, with the SAP particles deposited on the surface of the high loft layer being at least partially distributed and immobilized within the pores of the high loft layer. A tissue paper or a nonwoven is further adhesively attached on each of the high loft central layer to further immobilize the particles within the high loft central layer. At least the top cover layer which is planned to be oriented towards the topsheet on the absorbent article should be fluid-pervious. A further wrap layer may be typically used to further stabilize these layers and form the absorbent core. These absorbent cores are typically continuously produced as a unitary stream that can be collected in a roll. The roll can be transported to a diaper manufacturing line, where the absorbent cores are individualized by cutting and continuously assembled with the other components of the absorbent articles.

US 10,813,798, issued on October 27th 2020, describes the use of swellable absorbent fibers within the absorbent composite to increase fluid transfer and distribution within the composite leading to better intake and product fit. The swellable absorbent fibers provide this benefit in two ways: increased permeability and lower capillary pressure.

SUMMARY OF THE INVENTION

According to the present invention, a process for forming a composite absorbent structure is provided, the process comprising the steps of:

advancing a nonwoven web in a machine direction, wherein the nonwoven web has a first surface and an opposing second surface, wherein the first and second surfaces define a substrate comprising void spaces, and wherein the nonwoven web comprises from 20% to 100% by weight of superabsorbent fibres;
distributing superabsorbent particles on to the first surface of the nonwoven web;
and drawing at least some of the superabsorbent particles into void spaces of the nonwoven web substrate to form the composite absorbent structure.

The present invention further relates to composite absorbent structure comprising:
a first surface and an opposing second surface, wherein the first and second surfaces define a substrate comprising void spaces, and wherein the nonwoven web comprises from 20% to 100% by weight of superabsorbent fibres; wherein superabsorbent particles are distributed within at least some of the void spaces of the substrate and wherein the superabsorbent particles are distributed heterogeneously in the vertical direction between the first and second surfaces.

In another embodiment the present invention relates to absorbent article comprising a substantially liquid permeable topsheet, a substantially liquid impermeable backsheet, and a composite absorbent structure as described above, wherein the composite absorbent structure lies between the topsheet and the backsheet, and wherein the first surface is located on the side of the absorbent article closest to the topsheet, so that larger superabsorbent particles are predominantly distributed closest to a body side of the absorbent article.

High Loft Web

The term "high loft" refers to low density bulky fabrics, in contrast to flat, paper-like fabrics. High loft webs are characterized by a relatively high porosity. This means that there is a relatively high amount of void space in which superabsorbent polymer particles can be distributed.

The high loft layer (without the superabsorbent particles) of the invention may have a porosity of at least 90%, or at least 93%, or at least 95%, or at least 97%, as measured according to the porosity test method set out herein.

The high loft layer (without the superabsorbent particles) of the invention may have a density at a pressure of 4.14kPa (0.6 psi) below 0.20 g/cm$^3$, in particular ranging from 0.05 g/cm$^3$ to 0.15 g/cm$^3$.

The high loft layer (without the superabsorbent particles) of the invention may have a density at a pressure of 2.07 kPa (0.3 psi) below 0.20 g/cm$^3$, in particular ranging from 0.02 g/cm$^3$ to 0.15 g/cm$^3$.

The high loft layer (without the superabsorbent particles) of the invention may have a density at a pressure of 0.83 kPa (0.12 psi) below 0.15 g/cm$^3$, in particular ranging from 0.01 g/cm$^3$ to 0.15 g/cm$^3$.

The density can be calculated by dividing the basis weight of the high loft layer by its thickness measured at the respective pressure as indicated (see the method details further below in the "test procedure" section).

The thickness, basis weight and density of the high loft layer are typically homogenous in both transversal direction (x) and longitudinal direction (y). The orientation of fibers in the high loft layer may be non-homogenous such as predominant orientation of fibers into one direction x or y such as in carded nonwovens. Furthermore, the fiber orientation in the high loft layer in thickness direction z may be different versus the predominant orientation in one or both directions x and/or y.

The high loft layer may in particular have a thickness of at least 0.30 mm, in particular ranging from 0.3 mm to 3 mm, or from 0.5 mm to 2 mm, as measured at a pressure of 4.14 kPa (0.6 psi) (according to the test method described further below).

The high loft layer may in particular have a thickness ranging from 0.30 mm to 2.50 mm or from 0.5 to 2.0 mm or from 0.7 to 1.3 mm, as measured at a pressure of 0.83 kPa (0.12 psi) (according to the test method described further below).

The basis weight of the high loft layer may for example range from 15 gsm to 500 gsm, in particular from 50 gsm to 300 gsm, more particularly of from 75 gsm to 200 gsm.

The values indicated herein for the high loft layer are considered for the high loft material taken in isolation, that is before the SAP particles have been deposited between the fibers or an adhesive, if any, are applied to it, unless indicated otherwise.

High loft nonwoven layer

While the invention is not limited to a specific type of nonwoven layer, a preferred nonwoven layer is a needle-punched nonwoven web, also called "needlefelt". Needle-punched nonwoven webs are made from various fibrous webs (preferably carded needle-punched nonwoven webs) in which fibres are bonded together mechanically through fibre entanglement and frictions after fine needle barbs repeatedly penetrated through the fibrous web. Such needle-punched nonwoven webs provide low density, lofty structures which are particularly soft to touch, in part due to the absence of bonds within the structure.

An alternative example of suitable nonwoven layers are bonded carded webs ("BCW"). "Bonded carded web" refers to nonwovens that are made from staple fibers that are sent through a combing or carding unit, which separates and generally aligns the staple fibers in the machine direction to form a generally machine direction-oriented fibrous nonwoven web. This web can then be drawn through a heated drum, creating bonds throughout the fabric without applying specific pressure (through air bonding process). Such through air bonded carded web (TABCW) material provides a low density, lofty through-air bonded carded web. Through-air bonded carded web also have excellent bulk and compressibility, and rapid strike through and good rewet. Examples of suitable through air bonded carded webs are, for example, disclosed in WO2000/71067.

In a carded nonwoven, the fibers in the web are aligned predominantly in the machine direction and have a more uniform fiber alignment than other nonwovens, which results in greater stability and internal bond strength especially in machine direction. The carded nonwoven material can for example comprise about 3 to about 12 dtex staple fibers. In one particularly preferred example the superabsorbent fiber is 10 dtex and has a staple length of about 50 mm.

The bonding technique chosen, or the absence of bonding in the case of needle-punch nonwoven webs, influences the integrity of the fabric.

Superabsorbent Fibers

"Superabsorbent fiber" ("SAF") is used herein to refer to superabsorbent polymer material that is in a fibrous form. The superabsorbent fibers have a length and a cross-section. The length is the largest dimension of the fiber when the fiber is or would be laid flat and straight on a surface, such that curves or crimps in the fiber disappear and the fiber becomes an approximately rod-like form. The cross-section is orthogonal to the length. For purposes herein, a fiber is a material that has a largest dimension and smallest dimension, wherein the ratio of largest dimension to smallest dimension, preferably at least 15: 1, even more preferably at least 20: 1, i.e. the largest dimension of the superabsorbent fiber (also called the length) is at least 10 times, or at least 15 times, or at least 20 times the smallest dimension of the fiber (also called width). Preferably fibers are staple fibers and may have any cross-sectional shape such as circular, or substantially circular, cross-section. Superabsorbent fibers are preferably made from the same materials as superabsorbent polymers, further described below. Typical superabsorbent fibres are polyacrylate polymers, however it is not excluded that other polymer materials may also be used. For example, starch-based particulate absorbent polymer material may also be used, as well as polyacrylamide copolymer, ethylene maleic anhydride copolymer, cross-linked carboxymethylcellulose, polyvinyl alcohol copolymers, cross-linked polyethylene oxide, and starch grafted copolymer of polyacrylonitrile.

Superabsorbent fibers have a high capacity to absorb fluids, such as urine and other bodily fluids. The capacity of superabsorbent fibers to absorb an aqueous 0.9% saline solution may be measured using the Centrifuge Retention Capacity (CRC) test (EDANA method NWSP 241.0.R2 (19)), described herein below. The superabsorbent fibers may have a capacity of at least 7 g/g, or at least 8 g/g, or at least 10 g/g, or at least 15 g/g, or at least 18 g/g, or at least 20 g/g as measured according to the Centrifuge Retention Capacity test set out herein.

High loft nonwovens useful in the present invention may comprise from about 20% to about 100% by weight of superabsorbent fibers. Optionally, high loft nonwovens may comprise superabsorbent fibers in combination with from about 20% to about 80% of other synthetic fibers such as polyethylene, polypropylene, or bicomponent fibers. Suitable bicomponent fibers include polyester/polypropylene, PE/PP, bicomponent fibers. Preferred high loft nonwovens comprise from about 20% to about 80% by weight of superabsorbent fiber and from about 20% to about 80% by weight of bicomponent fiber.

Suitable nonwoven webs comprising superabsorbent fibers for use as the high loft layer of the present invention are commercially supplied by Technical Absorbents Ltd., Grimsby, UK (see exploresaf.com). Alternatively the high loft layer may be formed directly in line, for example by depositing staple fibres onto a continuous moving belt to provide the high loft layer which may be transported directly to the next processing step. Such a method of manufacturing absorbent fibrous structures is described in WO 2016/191439.

The high loft layer serves as substrate for the SAP particles which are at least partially distributed within void spaces of the porous structure. The SAP particles may be substantially uniformly blended across the thickness of the high loft layer. However, the SAP particles may be distributed heterogeneously in the vertical direction between the first and second surfaces. The SAP particles are typically deposited on one side of the nonwoven and drawn into the high loft nonwoven for example by gravity, by centrifugal force, by mechanical or air vibration (sound), by negative pressure (vacuum) on the opposite side of the nonwoven, or by any combination of these methods. In this way, some, typically larger, particles remain on and/or close to the surface of the high loft layer and other, typically smaller, particles may penetrate deeper within the void spaces of the high loft nonwoven structure. The SAP particles which are not trapped within the void spaces of the high loft layer but remain at the surface may optionally be further immobilized by a layer of adhesive. Adhesive, if any, may be applied on the top and bottom cover layers first before being combined while still tacky with the high loft central layer. SAP particles may be applied sequentially on the high loft layer from each side of the high loft layer as a first layer of SAP and a second layer of SAP. This process for SAP particles deposition may result in a SAP z-distribution pattern inside the central layer comprising two or more peaks of density separated by at least one buffer zone, when seen in the z direction.

Superabsorbent polymer particles

The term "superabsorbent polymer" (herein abbreviated as "SAP" in the singular and plural form) typically refers to absorbent materials that can absorb at least 7 times, and preferably at least 10 times, their weight of an aqueous 0.9% saline solution as measured using the Centrifuge Retention Capacity (CRC) test (EDANA method NWSP 241.0.R2 (19)), described herein below and referred herein as capacity. SAP are water-insoluble but water-swellable cross-linked polymers capable of absorbing large quantities of fluids. SAP are in particulate form so as to be flowable in the dry state. Typical particulate SAP are polyacrylate polymers, however it is not excluded that other polymer materials may also be used. For example, starch-based particulate absorbent polymer material may also be used, as well polyacrylamide copolymer, ethylene maleic anhydride copolymer, cross-linked carboxymethylcellulose, polyvinyl alcohol copolymers, cross-linked polyethylene oxide, and starch grafted copolymer of polyacrylonitrile.

SAP may be polyacrylates and polyacrylic acid polymers that are internally and/or surface cross-linked. The superabsorbent polymer of the invention may be selected from polyacrylates and polyacrylic acid polymers that are internally and surface cross-linked. The superabsorbent polymers can be internally cross-linked, i.e. the polymerization is carried out in the presence of compounds having two or more polymerizable groups which can be free-radically copolymerized into the polymer network. Exemplary superabsorbent polymer particles of the prior art are for example described in WO2006/083584, WO2007/047598, WO2007/046052, WO2009/155265, WO2009/155264. Preferably, the SAP particles comprise crosslinked polymers of polyacrylic acids or their salts or polyacrylates or derivatives thereof.

The SAP particles can at least partially penetrate within the pores of the high loft layers during the deposition step, so that they are at least partially distributed within the high loft central nonwoven. The particles are thus immobilized within the pores of the high loft layer on one hand, and by the tissue or nonwoven layer laminated on each of the top and bottom surface of the high loft central layer.

Some SAP particles, typically larger particles, remain at and/or close to the surface of the high loft layer and other, typically smaller, particles may penetrate deeper within the pores of the high loft nonwoven. It is preferred that the upper surface of the high loft layer, that is to say, the side of the high loft layer on to which the SAP is deposited, provides the upper surface or body-side surface of the absorbent core when the core is ultimately incorporated into an absorbent article such as a diaper or a feminine hygiene pad.

The Urine Permability Measurement (UPM) Test method typically measures the flow resistance of a preswollen layer of superabsorbent polymer particles, i.e. the flow resistance is measured at equilibrium. Therefore, such superabsorbent polymer particles having a high UPM value exhibit a high permeability when a significant volume of the absorbent article is already wetted by the liquid exudates. These embodiments exhibit good absorption properties not only at the first gush but also at the subsequent gushes.

The UPM permeability may be expressed in UPM value, where 1 UPM unit is $1 \times 10^{-7}$ $(cm^3.s)$ /g. The permeability of SAP is preferably more than 5 UPM units. This method is closely related to the SFC test method which has been used in some of the prior art. The Urine Permeability Measurement (UPM) Test Method is described in detail in WO 2016/106021, from page 21 to 28.

The permeability of SAP particles is preferably more than $6 \times 10^{-7}$ $cm^3.s/g$, or at least $7.5 \times 10^{-7}$ $cm^3.s/g$, or at least $10 \times 10^{-7}$ $cm^3.s/g$, or at least $15 \times 10^{-7}$ $cm^3.s/g$, preferably in the range of from $30 \times 10^{-7}$ $cm^3.s/g$ to $70 \times 10^{-7}$ $cm^3.s/g$

Superabsorbent material having the required properties may be sourced from commercial suppliers, which have a wide range of SAP property available. Typically capacity and permeability are in trade-off, as one SAP having high capacity may have relatively low permeability and vice-versa.

The SAP particles may be relatively small (under 1 mm in their longest dimension) in their dry state and may be roughly circular in shape, but granules, flakes, spheres, powders, platelets and other shapes and forms are also known to persons skilled in the art. Typically, the SAP may be in the form of spherical-like particles. The composite absorbent material may thus consist or consist essentially of the SAP distributed within the high loft nonwoven structure.

Some of SAP particles may be agglomerated, as e.g. taught in EP 3,391,961. The agglomerated superabsorbent polymer particles may be obtained by various methods. Agglomerated particles may be for example obtained by aggregating the precursor particles with an interparticle crosslinking agent reacted with the polymer material of the precursor particles to form crosslink bonds between the precursor particles have been for example disclosed in US 5,300,565, US 5,180,622, (both to Berg), US 5,149,334, US 5,102,597 (both to Roe), US 5,492,962 (Lahrman). Other ways to obtain agglomerated SAP particles are for example described in EP3056521 (Kim et al.), EP1512712 (Koji et al.), US10414876B2 (Jang et al.), US 7429009B2 (Nagasawa et al), EP220224911 (Higashimoto et al), EP2011803 (Handa et al).

Agglomerated superabsorbent polymer particles may also be obtained by a method comprising the steps of providing superabsorbent polymer particles and mixing the superabsorbent polymer particles with a solution comprising water and a multivalent salt having a valence of three or higher. This method is further disclosed EP2944376. The superabsorbent polymer particles of the core of the invention may in particular comprise at least 5%, or at least 10%, or at least 20%, or at least 30%, or at least 40%, or at least 50% by weight of the agglomerated superabsorbent polymer particles

The surface of the SAP particles may be coated. The surface of the SAP may be surface crosslinked. The SAP particles may also comprise surface and/or edge modified clay platelets. Preferably, the clay platelets are montmorillonite, hectorite, laponite or mixtures thereof. Preferably, the clay platelets are laponite. The SAP may comprise from 0.1 to 5% by weight of clay platelets with modified surfaces and/or edges compared to the weight of the precursor superabsorbent polymer particles.

SAP particles can also be characterized by the time it takes them to reach an uptake of 20 g/g, so-called T20. SAP particles having desired T20 can be synthesized using for example the teaching of WO2015/041,784A1 which discloses SAP having a T20 ranging of from 104 s to 211 s. The SAP particles having a desired T20 may also be acquired directly from conventional SAP suppliers offering a wide range of T20.

Unless otherwise indicated, the values indicated herein to qualify the SAP particles (e.g. CRC capacity, UPM permeability, AAP@0.7psi, T20...) refer to the properties of the SAP particles considered as raw material as it is supplied. These properties should be measured as much as possible before the absorbent core is manufactured as otherwise it may be difficult to separate the SAP particles from the finished absorbent core, as some of the larger particles may be partially glued to the top or bottom substrate layer, and the smaller particles may have moved further within the pores of the high loft layers.

The total amount of SAP particles present in the absorbent core may also vary according to expected user of the article. Diapers for newborns require less SAP particles than infant or adult incontinence diapers. The total amount of all SAP particles in the core may be for example comprised from about 2 g to 50 g, in particular from 5 g to 40 g, or for 10 g to 20 g for typical infant diapers. The basis weight for all SAP particles within the absorbent core may be for example of at least 50, 100, 200, 300, 400, 500 $g/m^2$ or more, or from 200 to 400 $g/m^2$. The average basis weight is calculated by dividing the weight of the SAP particles considered by the surface of the high loft central layer.

The absorbent core may typically comprise at least 60% by weight of superabsorbent polymer particles, preferably at least 70%, by total weight of the absorbent core.

Process

In an exemplary process for forming the composite absorbent structure the high loft layer and SAP particles are laid down on a moving surface and may be sandwiched between a top cover layer material and a bottom cover layer material. Each of the top and bottom cover layers is preferably paper, tissue or nonwoven web.

In a particularly preferred process the composite absorbent structure is formed on a rotating roll, referred to here as the lay-down roll. Firstly the top cover layer is unwound from a top cover layer web unwinder and the top cover layer is transferred to the lay-down roll. SAP particles are then supplied from an SAP particle dispenser and deposited onto the top cover layer. The SAP may be deposited onto the top cover layer either uniformly or non-uniformly in a pattern which is uneven in the machine direction or in the cross-machine direction, or in both directions. Methods of depositing SAP particles both uniformly and non-uniformly, in patterns, are known from the prior art.

The high loft layer, or optionally the high loft layer and the bottom cover layer, is placed on the lay-down drum so that the SAP particles is sandwiched between the top cover layer which is radially inside the SAP particles and the high loft layer which is radially outside the SAP particles. The bottom cover layer, if present, is the most radially outward layer on the lay-down drum. As the lay-down drum rotates, centrifugal forces acting on the SAP particles tend to urge the SAP particles radially outwards so that the SAP particles penetrates into the voids of the high loft layer.

Optionally other methods may be employed to promote the penetration of the SAP particles into the voids of the high loft layer such as mechanical vibration and/or sound.

Finally the composite absorbent structure comprising the top cover layer, SAP particles, high loft layer and, optionally, bottom cover layer is removed from the lay-down drum and may be subjected to further processing steps.

In the process embodiment in which the high loft layer is manufactured separately from the process of forming the composite absorbent structure, the high loft layer is typically brought to the manufacturing line in the form of pre-made rolls which are loaded onto an unwinder. Efficient transportation of pre-made rolls generally requires that the pre-made rolls are provided under at least some degree of compression. Therefore when pre-made rolls are unwound there may be a need to increase the void space by bulking up the high loft layer.

The high loft layer is unwound from the unwinder and advanced through the apparatus in a machine direction. For the purpose of the following process description the nonwoven web is advanced in a substantially horizonal plane having an upper surface (the first surface) and an opposing lower surface (the second surface). The upper surface of the high loft layer is disrupted to increase the void space of the high loft web in the region of the upper surface. In one embodiment of the invention the nonwoven web passes adjacent to a brush roll which is a roll, preferably a rotating roll, with protrusions such as spikes or bristles extending substantially radially outwardly from the roll surface. The protrusions engage with the fibrous region at the upper surface and lift at least some of the fibres thereby increasing the void space between the fibres. The speed of rotation of the brush roll may be synchronized so that the linear, circumferential speed of the protrusions is matched with the linear speed of the high loft web, but preferably the linear, circumferential speed of the protrusions is mismatched with the linear speed of the high loft web to optimally disrupt the upper surface of the high loft web and increase the void space. Preferably the linear, circumferential speed of the protrusions is at least 5% faster than the linear speed of the high loft web, more preferably at least 10% faster. Alternatively the circumferential speed of the protrusions may be at least 5% slower than the linear speed of the high loft web, more preferably at least 10% slower.

In another embodiment of the invention the surface disruption may be provided by a toothed roll in place of the brush roll. The toothed roll has teeth extending radially outwardly from the roll surface. The toothed roll disrupts the upper surface of the high loft web in the same way as described above for the brush roll.

In yet another embodiment of the invention the surface disruption may be provided by a comb, for example a toothed comb. The comb acts in a similar way to the rolls described above, except that the comb is substantially static.

More than one brush roll, or toothed roll, or comb, may be used in series to optimize the process of lofting the nonwoven web. For example 2, 4 or 6 rolls or combs may be used in series. The rolls or combs may be located to disrupt one surface only of the nonwoven web, the upper surface (first surface), or two or more rolls or combs may be located to disrupt both the upper surface (first surface) and the lower surface (second surface).

During the continuous process of making the absorbent cores, the top cover layer or the the bottom cover layer or both cover layers may pass through a spraying head so that glue is applied on one side of the cover layer before being attached to the high loft layer and/or SAP.

The composite absorbent structure may be consolidated by press rollers. The press rollers may have a substantially flat surface, or they may have elevated areas where extra pressure and heat should be applied onto the core. These elevated areas may coincide with the channel areas, and thus provide a mechanical bonding, ultrasonic bonding and/or heat bonding within the channel zones. The press rollers may be heated. It is also possible that the rollers have elevated areas along the longitudinal side edges and/or the back and front edges (360° perimeter) the core. A better bonding can be achieved in these zones when they are free of SAP particles as in the channel zones. Trimming knives can be provided to trim the longitudinal side edges of the continuous band of composite absorbent material before the stream of the composite absorbent material is finally rolled into a roll of composite absorbent material by the product roll winding roller.

The roll of composite absorbent material thus formed may be stored or transported to an article production site where it is further converted into an absorbent product. It is also possible that instead of forming a roll, the stream of absorbent core material may be directly fed into a converting line, in which case the absorbent cores will be individualized by cutting along their front and back edges.

A wrapping layer may also be fed before the composite absorbent material is rolled to wrap the top, central and bottom cover layer to prevent losses of SAP particles though the side edges of the composite absorbent core. Alternative such wrapping layer may also be attached to the core when further converting the core material web.

Absorbent article

The absorbent cores may be incorporated into any kind of personal hygiene articles, in particular pant diapers and taped diapers, as well as inserts in hybrid systems comprising a washable outer cover and a disposable insert. A schematic cross-sectional view showing some of the main components of a diaper absorbent article Absorbent articles typically comprise a wearer-facing fluid permeable topsheet and a garment-facing liquid impermeable backsheet attached to each other along their perimeter. The absorbent core is placed between these layers and may be attached directly and indirectly to these layers, typically by gluing or heat/pressure bonding.

The topsheet is preferably compliant, soft-feeling, and non-irritating to the wearer's skin. Further, at least a portion of the topsheet is liquid permeable, permitting liquids to readily penetrate through its thickness. A suitable topsheet may be manufactured from a wide range of materials, such as porous foams, reticulated foams, apertured plastic films, or woven or nonwoven materials of natural fibers (e.g., wood or cotton fibers or viscose), synthetic fibers or filaments (e.g., polyester or polypropylene or bicomponent PE/PP fibers or mixtures thereof), or a combination of natural and synthetic fibers. If the topsheet includes fibers, the fibers may be spunbond, carded, wet-laid, meltblown, hydroentangled, or otherwise processed as is known in the art, in particular spunbond PP nonwoven. Typical diaper topsheets have a basis weight of from about 10 gsm to about 28 gsm, in particular between from about 12 gsm to about 18 gsm but other basis weights are possible.

The backsheet is typically impermeable to liquids (e.g. urine). The backsheet may for example be or comprise a thin plastic film such as a thermoplastic film having a thickness of less than about 0.10 mm. Exemplary backsheet films include those manufactured by Tredegar Corporation, based in Richmond, VA, and sold under the trade name CPC2 film. Other suitable backsheet materials may include breathable materials which permit vapors to escape from the article while still preventing exudates from passing through the backsheet. A covering low basis weight nonwoven may be attached to the external surface of the film to provide for a softer touch.

The absorbent articles may also comprise a liquid management layer (also called fluid acquisition or fluid distribution layer) directly under the topsheet. The function of such a layer is to rapidly acquire the fluid from the topsheet away from the wearer-facing side and/or to distribute over a larger area so it is more efficiently absorbed by the absorbent core. It is also possible that such a liquid management layer may be placed between the backsheet and the absorbent core. A further layer may be present between the liquid management and the absorbent core. The further layer may be another such acquisition or distribution layer, or may be a tissue paper or low basis weight NW layer that provides an additional wrapping of the absorbent core to avoid SAP particles from escaping outside the core.

Absorbent articles such as diapers or training pants may typically further comprise components that improve the fit of the article around the legs of the wearer, in particular barrier leg cuffs and gasketing cuffs. The barrier leg cuffs may be formed by a piece of material, typically a nonwoven, which is partially bonded to the rest of the article and can be partially raised away and thus stand up from the plane defined by the topsheet. The barrier leg cuffs are typically delimited by a proximal edge joined to the rest of the article, typically the topsheet and/or the backsheet, and a free terminal edge intended to contact and form a seal with the wearer's skin. The standing up portion of the cuffs typically comprise an elastic element, for example one or a plurality of elastic strands. The barrier leg cuffs provide improved containment of liquids and other body exudates approximately at the junction of the torso and legs of the wearer.

In addition to the barrier leg cuffs, the article may comprise gasketing cuffs, which are formed in the same plane as the chassis of the absorbent article, in particular which may be at least partially enclosed between the topsheet or the barrier leg cuffs and the backsheet, and may be placed laterally outwardly relative to the upstanding barrier leg cuffs. The gasketing cuffs can provide a better seal around the thighs of the wearer. Usually each gasketing leg cuff will comprise one or more elastic string or elastic element comprised in the chassis of the diaper for example between the topsheet and backsheet in the area of the leg openings.

The absorbent articles may also include other typical components found in diapers, training pants, replaceable inserts or adult incontinence products (and not further represented). A releasable fastening system for taped diapers may be provided to provide lateral tensions about the circumference of the absorbent article to hold the absorbent article on the wearer. This fastening system is not necessary for training pants since the waist region of these articles is already bonded. The fastening system usually comprises a fastener such as tape tabs, hook and loop fastening components, interlocking fasteners such as tabs & slots, buckles, buttons, snaps, and/or hermaphroditic fastening components, although any other known fastening means are generally acceptable. A landing zone is normally provided on the front waist region of the article for the fastener to be releasably attached.

The absorbent article may comprise front ears and back ears as is known in the art. The ears can be integral part of the chassis, for example formed from the topsheet and/or backsheet as side panel. Alternatively, they may be separate elements attached by gluing and / or heat embossing. The back ears are advantageously stretchable to facilitate the attachment of the tabs on the landing zone and maintain the taped diapers in place around the wearer's waist. The front ears may also be elastic or extensible to provide a more comfortable and contouring fit by initially conformably fitting the absorbent article to the wearer and sustaining this fit throughout the time of wear well past when absorbent article has been loaded with exudates since the elasticized ears allow the sides of the absorbent article to expand and contract.

Typically, adjacent layers will be joined together using conventional bonding method such as adhesive coating via slot coating or spraying on the whole or part of the surface of the layer, or thermo-bonding, or pressure bonding or combinations thereof. Adjacent layers of the article should be considered to be attached to another unless specifically mentioned otherwise. For example the backsheet and the bottom cover layer of the absorbent core may be typically glued together. The adhesives used may be any standard hotmelt glue as known in the art.

Test Methods

Centrifuge Retention Capacity (CRC)

The CRC measures the capacity of the superabsorbent polymer particles to absorb for free swelling in excess liquid. The CRC is measured according to EDANA method NWSP 241.0.R2 (19).

In deviation from EDANA NWSP 241.0.R2(19) the sampling (chapter 8 in EDANA NWSP 241.0.R2(19)) for the superabsorbent fibers, superabsorbent nonwovens and/or the superabsorbent core is as following:
The superabsorbent fibers, superabsorbent nonwovens and/or the superabsorbent core are cut into pieces with approximately 5 mm as largest dimension. The cutting can e.g. be done manually with scissors. Care is taken that the fibrous structure (the core, the nonwoven or the bulk of fibers) is not majorly compressed before or during the cutting process. This ensures sufficient void space between the superabsorbent fibers, so they can be predominately wetted by the swelling medium at the entire surface area.

Further deviations from or additions to EDANA NWSP 241.0.R2(19) in the procedure (chapter 9.1-9.5 in EDANA NWSP 241.0.R2(19)) for the superabsorbent fibers, superabsorbent nonwovens and/or the superabsorbent core are as following:
The sample for the measurement is taken carefully, e.g. with a lab pincet, to put it into the teabag. With a lab pincet, the fibers are carefully distributed in the teabag to avoid lumps and fiber lumps, if any, are carefully opened.

When sealing the teabag, care is taken that no material of the superabsorbent fibers, superabsorbent nonwovens and/or the superabsorbent core is in the area of the seal. This ensures a complete and sufficiently strong sealing of the teabag.

All other items of the test method are executed as set out in EDANA NWSP 241.0.R2(19).

Absorption Against Pressure

The AAP is measured according to EDANA standard test NWSP 242.0 R2 (19), with the pressure used being 0.7 psi, as indicated as AAP@0.7psi.

Thickness and Density Measurement Method

This method is used to measure the thickness (caliper) of the layer in a standardized manner. The density can then be calculated from the thickness and the basis weight of the layer. Unless otherwise mentioned, the thickness and density are indicated for the high loft material in the absence of SAP particles. The measurement should preferably be made on the high loft material before it was converted into an absorbent core and thus free of SAP particles. If the starting material is not available, the high loft central layer can be obtained by carefully extracting it from an absorbent core, and removing the majority of SAP particles for example by careful shaking or suction. A freeze spray may be used to separate the central layer from the other layers. The samples should be kept at least 24 hours at 21°C $\pm$ 2°C and 50% $\pm$ 10% RH to equilibrate, in particular if they have been previously compressed.
Equipment: Mitutoyo manual caliper gauge with a resolution of 0.01 mm, or equivalent instrument.

Contact Foot: Flat circular foot with a diameter of 16.0 mm ($\pm$ 0.2 mm). A circular weight may be applied to the foot (e.g., a weight with a slot to facilitate application around the instrument shaft) to achieve the target weight. The total weight of foot and added weight (including shaft) is selected to provide the desire pressure, for example 4.14kPa of pressure (0.6 psi) to the sample. The thickness can be determined at different pressures, using accordingly different weights applied to the foot. The thickness and density measurements indicate the applied pressure, for example measured at 4.14 kPa (0.6 psi) or 1.2 kPa.

The caliper gauge is mounted with the lower surface of the contact foot in a horizontal plane so that the lower surface of the contact foot contacts the center of the flat horizontal upper surface of a base plate approximately 20 x 25 cm. The gauge is set to read zero with the contact foot resting on the base plate.
Ruler: Calibrated metal ruler graduated in mm.
Stopwatch: Accuracy 1 second.
Sample preparation: The central layer is conditioned at least 24 hours as indicated above.

Measurement procedure: The layer is laid flat with the bottom side, i.e. the side intended to be placed towards the backsheet in the finished article facing down. The point of measurement, i.e. the middle of the sample, is carefully drawn on the top side of the layer, taking care not to compress or deform the layer. In the unlikely case that the high loft nonwoven layer is not homogeneous in the transversal direction or longitudinal direction, the values are measured in the center of a sample corresponding to the center of an absorbent core that would be made from the sample.

The contact foot of the caliper gauge is raised and the central layer is placed flat on the base plate of the caliper gauge with the top side of the core up so that when lowered, the center of the foot is on the marked measuring point.

The foot is gently lowered onto the sample and released (ensure calibration to "0" prior to the start of the measurement). The caliper value is read to the nearest 0.01 mm, 10 seconds after the foot is released.

The procedure is repeated for each measuring point. Ten samples are measured in this manner for a given material and the average thickness is calculated and reported with an accuracy of one tenth mm. The basis weight of each sample is calculated by dividing the weight of each sample by their area.

The density, in g/cm$^3$, is calculated by dividing the basis weight (in g/cm$^2$) of the material by the thickness (in cm).

Porosity Test Method

Porosity can be calculated from basis weight, caliper, and specific fiber density. Porosity can be calculated with:

$$n = 1 - \frac{BW}{d \cdot \rho_{fib}}$$

wherein
$\rho_{fib}$ is the density of the fibers
d is the caliper of the nonwoven web

BW is the basis weight of the web
n is the porosity

The density $\rho_{fib}$ of the fibers can be determined by pycnometry with a suitable non-swelling liquid of known density.

The caliper d is measured with the dry absorbent core caliper test method above.

Basis weight (BW) is determined by the test method described above.

SAP K(t) Test Method

This method determines the time dependent effective permeability SAP K(t) and the uptake kinetics of a gel layer formed from hydrogel-forming superabsorbent polymer particles or of an absorbent structure containing such particles under a confining pressure. The objective of this method is to assess the ability of the gel layer formed from hydrogel-forming superabsorbent polymer particles or the absorbent structure containing them to acquire and distribute body fluids when the polymer is present at high concentrations in an absorbent article and exposed to mechanical pressures as they typically occur during use of the absorbent article. Darcy's law and steady-state flow methods are used to calculate effective permeability (see below). (See also for example, "Absorbency," ed. By P.K. Chatterjee, Elsevier, 1982, Pages 42-43 and "Chemical Engineering Vol. II, Third Edition, J.M. Coulson and J.F. Richardson, Pergamon Press, 1978, Pages 122-127.)

In contrast to previously published methods, the sample is not preswollen therefore the hydrogel is not formed by preswelling hydrogel-forming superabsorbent polymer particles in synthetic urine, but the measurement is started with a dry structure. The equipment used for this method is called 'Zeitabhängiger Durchlässigkeitsprüfstand' or 'Time Dependent Permeability Tester', Equipment No. 03-080578 and is commercially available at BRAUN GmbH, Frankfurter Str. 145, 61476 Kronberg, Germany and is described below. Upon motivated request, operating instructions, wiring diagrams and detailed technical drawings are also available. A detailed description of the equipment and principle is further described in WO2015/041784 (P&G).

The SAP K(t) Test Method can be in particular used to measure the time required to reach an uptake of 20 g/g, starting at 0 s (t0) in s, the so-called T20. Using the test method, the time required to reach a certain uptake can be determined by linear interpolation. The time where the uptake of 20 g/g is first reached is called T20. The average values for T20 are reported from 3 replicates according to the accuracy required as known by the skilled man.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

**Claims**

1. A process for forming a composite absorbent structure, the process comprising the steps of:

   advancing a nonwoven web in a machine direction, wherein the nonwoven web has a first surface and an opposing second surface, wherein the first and second surfaces define a substrate comprising void spaces, and wherein the nonwoven web comprises from 20% to 100% by weight of superabsorbent fibres;
   distributing superabsorbent particles on to the first surface of the nonwoven web; and
   drawing at least some of the superabsorbent particles into void spaces of the nonwoven web substrate to form the composite absorbent structure.

2. The process according to claim 1 wherein the nonwoven web is a high loft web having a density at a pressure of 0.83 kPa (0.12 psi) of less than 0.20 g/cm$^3$.

3. The process according to either of claims 1 or 2 wherein the nonwoven web is a needle-punched nonwoven web, preferably a carded, needle-punched nonwoven web.

4. The process according to either of claims 1 or 2 wherein the nonwoven web is a bonded carded nonwoven web, preferably a through-air bonded carded nonwoven web.

5. The process according to any of claims 1 to 4 wherein the nonwoven web comprises from 20% to 80% by weight of superabsorbent fibers and from 20 to 80% of other synthetic fibers, preferably the nonwoven web comprises from 20% to 80% by weight of superabsorbent fibers and from 20 to 80% of bicomponent fibers.

6. The process according to any of the previous claims wherein at least the first surface of the nonwoven web is disrupted to increase the void space of the nonwoven web in the region of the first surface by applying a brush roll, toothed wheel or comb to the first surface.

7. The process according to any of the previous claims wherein the step of drawing at least some of the superabsorbent particles into void spaces of the nonwoven web substrate comprises the step of applying centrifugal forces, mechanical vibration, sound, vacuum, or applying any combination of those forces.

8. A composite absorbent structure comprising:

   a first surface and an opposing second surface, wherein the first and second surfaces define a substrate comprising void spaces, and wherein the nonwoven
   web comprises from 20% to 100% by weight of superabsorbent fibres;;

   wherein superabsorbent particles are distributed within at least some of the void spaces of the substrate and wherein the superabsorbent particles are distributed heterogeneously in the vertical direction between the first and second surfaces.

9. The composite absorbent structure according to claim 8 wherein larger superabsorbent particles are predominantly distributed close to the first surface of the substrate and smaller superabsorbent particles penetrate deeper within the void spaces of the substrate of the nonwoven web.

10. The composite absorbent structure according to either of claims 8 or 9 wherein the nonwoven web is a high loft web having a density at a pressure of 0.83 kPa (0.12 psi) of less than 0.20 g/cm$^3$.

11. The composite absorbent structure according to any of claims 8 to 10 wherein the nonwoven web is a needle-punched nonwoven web, preferably a carded, needle-punched nonwoven web.

12. The composite absorbent structure according to any of claims 8 to 10 wherein the nonwoven web is a bonded carded nonwoven web, preferably a through-air bonded carded nonwoven web.

13. The composite absorbent structure according to any of claims 8 to 12 wherein the nonwoven web comprises from 20% to 80% by weight of superabsorbent fibers and from 20 to 80% of other synthetic fibers, preferably the nonwoven web comprises from 20% to 80% by weight of superabsorbent fibers and from 20 to 80% of bicomponent fibers.

14. An absorbent article comprising a substantially liquid permeable topsheet, a substantially liquid impermeable backsheet, and a composite absorbent structure according to any of claims 9 to 13, wherein the composite absorbent structure lies between the topsheet and the backsheet, wherein the first surface is located on the side of the absorbent article closest to the topsheet, so that larger superabsorbent particles are predominantly distributed closest to a body side of the absorbent article.

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2018/193517 A1 (FELDKAMP JOSEPH R [US] ET AL) 12 July 2018 (2018-07-12) * abstract * * paragraphs [0003] - [0007], [0026], [0029], [0039] - [0054] * | 1-14 | INV. A61F13/15 A61F13/532 A61F13/53 |
| X | US 2009/099541 A1 (QIN JIAN [US] ET AL) 16 April 2009 (2009-04-16) * figures 6-8 * * paragraphs [0011] - [0014], [0108], [0116], [0126] - [0187] * | 1-14 | |
| A | EP 0 339 461 A1 (KIMBERLY CLARK CO [US]) 2 November 1989 (1989-11-02) * abstract * * page 3, lines 17-19 * * page 4, lines 12-32 * * example I and II * | 1-14 | |
| A | WO 2015/002934 A2 (DSG TECHNOLOGY HOLDINGS LTD; WRIGHT ANDREW [GB] ET AL.) 8 January 2015 (2015-01-08) * paragraphs [00107], [00142], [00150], [00155] * | 1-14 | TECHNICAL FIELDS SEARCHED (IPC) A61F |

The present search report has been drawn up for all claims

| Place of search | Date of completion of the search | Examiner |
|---|---|---|
| Munich | 8 October 2021 | Schmitt-Humbert, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 17 2970

This annex lists the patent family members relating to the patent documents cited in the above-mentioned European search report.
The members are as contained in the European Patent Office EDP file on
The European Patent Office is in no way liable for these particulars which are merely given for the purpose of information.

08-10-2021

| Patent document cited in search report | | Publication date | Patent family member(s) | | Publication date |
|---|---|---|---|---|---|
| US 2018193517 | A1 | 12-07-2018 | US | 2018193517 A1 | 12-07-2018 |
| | | | WO | 2017019176 A1 | 02-02-2017 |
| US 2009099541 | A1 | 16-04-2009 | AU | 2008313369 A1 | 23-04-2009 |
| | | | BR | PI0816529 A2 | 23-04-2009 |
| | | | CN | 101827616 A | 08-09-2010 |
| | | | EP | 2207576 A2 | 21-07-2010 |
| | | | JP | 5260663 B2 | 14-08-2013 |
| | | | JP | 2011500124 A | 06-01-2011 |
| | | | KR | 20100083147 A | 21-07-2010 |
| | | | US | 2009099541 A1 | 16-04-2009 |
| | | | WO | 2009050613 A2 | 23-04-2009 |
| EP 0339461 | A1 | 02-11-1989 | AU | 609598 B2 | 02-05-1991 |
| | | | CA | 1321062 C | 10-08-1993 |
| | | | DE | 68904461 T2 | 19-05-1993 |
| | | | EP | 0339461 A1 | 02-11-1989 |
| | | | ES | 2053855 T3 | 01-08-1994 |
| | | | MX | 170214 B | 11-08-1993 |
| | | | ZA | 892846 B | 27-12-1989 |
| WO 2015002934 | A2 | 08-01-2015 | AU | 2014284437 A1 | 11-02-2016 |
| | | | AU | 2019204336 A1 | 11-07-2019 |
| | | | CA | 2916628 A1 | 08-01-2015 |
| | | | CN | 105682624 A | 15-06-2016 |
| | | | CN | 113230030 A | 10-08-2021 |
| | | | EP | 3016624 A2 | 11-05-2016 |
| | | | HK | 1225596 A1 | 15-09-2017 |
| | | | IL | 243453 A | 29-10-2020 |
| | | | JP | 6745719 B2 | 26-08-2020 |
| | | | JP | 2016526983 A | 08-09-2016 |
| | | | JP | 2019205917 A | 05-12-2019 |
| | | | KR | 20160043955 A | 22-04-2016 |
| | | | MX | 362615 B | 28-01-2019 |
| | | | NZ | 716077 A | 27-11-2020 |
| | | | PH | 12015502795 A1 | 14-03-2016 |
| | | | PL | 3016624 T3 | 31-01-2020 |
| | | | RU | 2016102681 A | 08-08-2017 |
| | | | SG | 10201800994R A | 28-03-2018 |
| | | | SG | 11201510306V A | 28-01-2016 |
| | | | US | 2015045756 A1 | 12-02-2015 |
| | | | US | 2019167493 A1 | 06-06-2019 |
| | | | WO | 2015002934 A2 | 08-01-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9511654 A
- WO 2008155699 A
- WO 2016106021 A
- US 10813798 B
- WO 200071067 A
- WO 2016191439 A
- WO 2006083584 A
- WO 2007047598 A
- WO 2007046052 A
- WO 2009155265 A
- WO 2009155264 A
- EP 3391961 A
- US 5300565 A
- US 5180622 A, Berg
- US 5149334 A
- US 5102597 A, Roe
- US 5492962 A, Lahrman
- EP 3056521 A, Kim
- EP 1512712 A, Koji
- US 10414876 B2, Jang
- US 7429009 B2, Nagasawa
- EP 220224911 A, Higashimoto
- EP 2011803 A, Handa
- EP 2944376 A
- WO 2015041784 A1
- WO 2015041784 P

### Non-patent literature cited in the description

- Absorbency. Elsevier, 1982, 42-43
- **J.M. COULSON ; J.F. RICHARDSON.** Chemical Engineering. Pergamon Press, 1978, vol. II, 122-127